# EUROPEAN PATENT APPLICATION

(11) **EP 1 709 991 A1**
(43) Date of publication of application: **11.10.2006**
(21) Application number: 05704255.8
(22) Date of filing: 28.01.2005
(51) Int. Cl.: A61N 1/32, A61N 1/04

(54) **ELECTRODE FOR TREATMENT, AND TREATMENT DEVICE**

(30) Priority: 30.01.2004 JP 2004024900
(71) Applicant: Yaman Ltd., Tokyo 135-0045 (JP)
(72) Inventor: YAMAZAKI, Iwao, c/o YA-MAN LTD.,, Tokyo 1350045 (JP)
(74) Representative: Maury, Richard Philip
(86) International application number: PCT/JP2005/001257
(87) International publication number: WO 2005/072820

(57) **Abstract**

A metal backer forming device (1), comprising a film withdrawing roller (2) for withdrawing, starting at one end, a transfer film (F) from the wound body of the transfer film (F) formed by applying at least a metal film (26a) onto a base film (26b), a film carrying mechanism such as a turn roller (9) for carrying the transfer film (F) withdrawn from the wound body to the downstream side, a transfer roller (12) for transferring the metal film (26a) by heating while pressing the carried transfer film (F) against a phosphor screen (22) installed on a face plate (27), and a film winding roller (18) for winding while releasing the base film (26b) from the transfer film (F) which completes transfer treatment.

## Description

### TECHNICAL FIELD

The present invention relates to an electrode and a device for slimming treatment, beautiful skin treatment and the like.

### BACKGROUND ART

In a human body, a bioelectric current is always flowed so as to maintain the human body activity so that the cellular activity and the muscle contracting-relaxing activity can be maintained. In this point of view, if an external weak current is flowed on purpose in the human body to be stimulated, the cellular activity is stimulated and thus, the muscle contracting-relaxing activity is also stimulated so as to enhance the vital function as the bioelectric current is flowed in the human body.

Therefore, such a pulsed current-type health device for various treatments such as fatigue recovery, muscle pain recovery, body slimming or beautiful skinning as supplying a weak pulsed current to a human body from electrodes attached to the skin of the human body is known (refer to Patent Document 1).

With the above-mentioned conventional device as described in Patent Document 1, the handling performance of the device is deteriorated because the device includes the cables to connect the electrodes to be contacted to the human body and the electric power source, so that it is desired that the handling performance of the device can be developed through the improvement of the cable.
Patent Document 1: JP-A 2002-292912 (KOKAI)

### DISCLOSURE OF THE INVENTION

The present invention is established so as to iron out the above-mentioned problem, and thus, it is an object of the present invention to provide an electrode and device for various treatments through the enhancement of the handling performance.

In order to achieve the object, this invention relates to an electrode for treatment including an electric power source; a conducting pad for attaching a human body; a treatment current supplying means for supplying a pulsed current to a body part to which the conducting pad is attached on the electric power supply from the electric power source; a receiving means for receiving an external control signal at radio transmission; and a controlling means for controlling the treatment current supplying means on the basis of the control signal received by the receiving means at radio transmission. According to the present invention, since the pulsed current can be supplied to the human body under the cordless condition, the operationality for parts and instruments a user should handle can be developed remarkably at treatment and thus, the user can realize the intended treatment such as slimming treatment easily.

In the treatment electrode according to the present invention, the treatment current supplying means is configured so as to stop the supply of said pulsed current at a given period. In this case, since the pulsed current stop period is formed, the muscle of the human body can be rest so that the muscle training efficiency by the successive pulsed current supply after a given periodic rest can be developed.

In the treatment electrode according to the present invention, a plurality of conducting pads may be prepared. In this case, the treatment electrode can conduct a predetermined treatment for a body part which is located between the conducting pad. In this embodiment, a conducting pad connector for electrically and mechanically connecting the plurality of pads may be prepared so that the length of the conducting pad connector is changeable. Since the distance between the conducting pads can be easily controlled, the position of the body part to be treated can be shifted simply. Instead, the conducting pad connector may be flexible. In this case, since the electrode connector can be deformed commensurate with the convex-concave surface shape of the human body, the conducting pads can be attached to the surface region of the human body steadily so that the intended treatment process can be conducted.

In the treatment electrode according to the present invention, an impedance measuring means for measuring the impedance of the body part to which the conducting pad is attached by flowing a measuring current in the body part may be provided. In this case, a given impedance as information source necessary for gaining the body fat, the muscle bulk, the bone mass and/or the water content of the body part can be obtained.

In the treatment electrode according to the present invention, an impedance information transmitting means for transmitting a measurement result in impedance by the impedance measuring means outside at radio transmission may be provided so that the receiving means receives, as the control signal, treatment controlling information corresponding to the body fat, the muscle bulk, the bone mass and/or the water content of the body part which are calculated from the measured impedances. In the treatment electrode according to the present invention, the controlling means varies the output and/or frequency of the pulsed current to be supplied to the human body by the treatment current supplying means on, as the control signal, the treatment controlling information corresponding to the body fat, the muscle bulk, the bone mass and/or the water content of the body part. In this case, the optimum treatment can be conducted for the body part about which the body fat or the like is known on the measured body fat or the like.

In the treatment electrode according to the present invention, the controlling means varies the width of the pulsed current commensurate with the degree of the body fat of the body part which is calculated. In this case, the response period of muscle against electric stimulation is longer at a body part with higher body fat and shorter at a body part with lower body fat. In this point of view, the pulsed current with longer pulse width is supplied to the body part with more body fat and the pulsed current with shorter pulse width is supplied to the body part with less body fat. As a result, since an appropriate pulsed current can be supplied to the body part in accordance with the body fat of the body part, the sliming treatment can be conducted effectively and efficiently.

In the treatment electrode according to the present invention, a pad adhering means for adhering the conducting pad to the human body may be provided. As the pad adhering means, a belt which is configured such that the conducting pad is pressed against the skin surface of the human body can be exemplified. The pad adhering means may be configured such that the conducting pad is made of an adhesive sheet with electric conduction such as gel sheet. In the latter case, the adhesion between the conducting pad and the skin surface can be enhanced without any holding means. Therefore, with the use of the simplified treatment electrode, the efficiency of the pulsed current supply, i.e., the efficiency of the treatment can be enhanced.

In the treatment electrode according to the present invention, the conducting pad is comprised of a plurality of conducting pads commensurate with different kinds of shapes of the body part. In this case, since the exclusively shaped conducting pad can be applied to the body part to be treated, the treatment for the body part can be conducted appropriately.

In the treatment electrode according to the present invention, a clothing with the plurality of conducting pads which are fixed so as to be contacted to the body part at treatment may be provided. In this case, when a user puts the clothing on, the conducting pads for exclusive use are contacted to the body parts to be treated, so that the setting of the conducting pads before treatment can be simplified.

In the treatment electrode according to the present invention, a number of heart beat detecting means for detecting the number of heart beat through the conducting pad to be contacted to the body part may be provided. In this case, the treatment mode can be varied on the detected number of heart beat.

In order to achieve the object, this invention relates to a device for treatment comprises a treatment electrode, and a controller for controlling the treatment electrode, and the controller includes an input means for inputting information about the treatment, a control signal generating means for generating a control signal on the information input by the input means, and a control signal transmitting means for transmitting the control signal generated by the control signal generating means at radio transmission.

In the treatment device according to the present invention, when a given treatment information such as treatment mode is input into the device via the controller, the control signal corresponding to the treatment information is transmitted at radio transmission. At the treatment electrode, the pulsed current supply corresponding to the control signal received at radio transmission is conducted to at least one pair of conducting pads to be contacted to the body part. According to this aspect of the present invention, therefore, the handling of the treatment device can be simplified so that the intended treatment can be conducted easily. Herein, the treatment mode can be set by changing the output and/or the frequency of the pulsed current to be supplied from the pulsed current supplying section.

In the treatment device according to the present invention, a plurality of conducting pads may be provided. Then, a pulsed current supply selecting means which is configured so as to switchably select a pair of conducting pads or a pair of treatment electrodes for supplying a pulsed current through a treatment current supplying means from among three or more conducting pads or treatment electrodes may be provided. In these cases, the intended treatment can be conducted to the body part substantially sandwiched between the conducting pads (e.g., the conducting pads of the treat electrode) without the shift of the conducting pads on the human body.

In the treatment device according to the present invention, the pulsed current supply selecting means is configured so as to switchably and successively select the pair of conducting pads or the pair of treatment electrodes so that the pulsed current is supplied successively through the successively selected pair of conducting pads or treatment electrodes. In this case, the contraction and relaxation of muscle around the body part to be treated due to the electric stimulation through the conducting pad can not be compensated so that the sliming treatment can not be compensated.

In the treatment device according to the present invention, the controller includes a selecting means which is configured so as to switchably select a pair of conducting pads or a pair of treatment electrodes for measuring an impedance of the body part through an impedance measuring means from among three or more conducting pads or treatment electrodes. In this case, the intended treatment can be conducted to the body part substantially sandwiched between the conducting pads (e.g., the conducting pads of the treat electrode) without the shift of the conducting pads on the human body.

In the treatment device according to the present invention, the controller includes a selecting means which is configured so as to switchably select a pair of conducting pads or a pair of treatment electrodes for measuring an impedance of the body part through an impedance measuring means from among three or more conducting pads or treatment electrodes. In this case, the intended treatment can be conducted to the body part substantially sandwiched between the conducting pads (e.g., the conducting pads of the treat electrode) without the shift of the conducting pads on the human body, and the impedance of the body part sandwiched by the conducting pads can be obtained.

In the treatment device according to the present invention, the treatment electrode further includes an impedance measuring means for measuring the impedance of the body part to which the conducting pad is attached by flowing a measuring current in the body part, and the controller further includes: an impedance information receiving means for receiving, at radio transmission, an impedance measurement result of the body part which is measured by the impedance measuring means; a calculating means for calculating the body fat, the muscle bulk, the bone mass and/or the water content of the body part on the measured impedance received by the impedance information receiving means; a control signal generating means for generating treatment information as a control signal on the calculated result by the calculating means; and a control signal transmitting means for transmitting, at radio transmission, the control signal generated by the control signal generating means. In this case, since the treatment mode can be varied on the body fat, the muscle bulk, the bone mass and/or the water content of the body part, the optimum treatment can be conducted for the user.

### BRIEF DESCRIPTION OF THE DRAWINGS

[Fig. 1] Fig. 1 is a structural view schematically showing a treatment device according to a first embodiment of the present invention.
[Fig. 2] Fig. 2 is a structural view schematically showing the treatment electrode of the treatment device as shown in Fig. 1.
[Fig. 3] Fig. 3 is a cross sectional view of the treatment electrode as shown in Fig. 2.
[Fig. 4] Fig. 4 is a block diagram functionally showing the construction of the treatment device as shown in Fig. 1.
[Fig. 5] Fig. 5 is a block diagram functionally showing the construction of the pulsed current-supplying section of the treatment device as shown in Fig. 1.
[Fig. 6] Fig. 6 is a structural view showing another type of treatment electrode to be equipped to the treatment device as shown in Fig. 1.
[Fig. 7] Fig. 7 is an elevational view showing a clothing equipped with the treatment electrode according to the second embodiment of the present invention.
[Fig. 8] Fig. 8 is a backside view of the clothing as shown in Fig. 7.
[Fig. 9] Fig. 9 is a structural view showing a treatment device according to third embodiment of the present invention.
[Fig. 10] Fig. 10 is a structural view of the treatment electrode equipped to the treatment device as shown in Fig. 9.
[Fig. 11] Fig. 11 is a structural view showing another type of treatment electrode which is different from the treatment electrode as shown in Fig. 10.
[Fig. 12] Fig. 12 is a structural view showing the liquid crystal displaying section of the controller equipped to the treatment device as shown in Fig. 9.
[Fig. 13] Fig. 13 is a structural view showing the matrix displaying region of the liquid crystal displaying section.
[Fig. 14] Fig. 14 is a plan view of the controller in Fig. 12.
[Fig. 15] Fig. 15 is a side view of the controller in Fig. 12.
[Fig. 16] Fig. 16 is a backside view of the controller in Fig. 12.
[Fig. 17] Fig. 17 is a schematic view showing the entire controlling operation by the treatment device as shown in Fig. 9.
[Fig. 18] Fig. 18 is a Table relating to the combination of a pair of electrodes among six treatment electrodes which are attached to the human body.
[Fig. 19] Fig. 19 is a schematic view showing some combinations of electrodes on the human body.
[Fig. 20] Fig. 20 is a schematic view showing other combinations of electrodes on the human body.
[Fig. 21] Fig. 21 is a Table showing a treatment course carried out by the treatment device as shown in Fig. 9.
[Fig. 22] Fig. 22 is a schematic view showing a wave form of a pulsed current to be supplied to the human body through the treatment electrode of the treatment device as shown in Fig. 9.
[Fig. 23] Fig. 23 is a schematic view showing wave forms of pulsed currents to be supplied to the human body through the treatment electrodes of the treatment device as shown in Fig. 9.
[Fig. 24] Fig. 24 is a flow chart relating to the transmission between the controller and the treatment electrodes of the treatment device as shown in Fig. 9.
[Fig. 25] Fig. 25 is a perspective view showing the treatment electrode of a treatment device according to a fourth embodiment of the present invention.
[Fig. 26] Fig. 26 is a perspective view showing the treatment electrode as shown in Fig. 25, as viewed from the terminal for electric charge.
[Fig. 27] Fig. 27 is a plan view showing the treatment electrode as viewed from the conductive pad and showing the gel sheet applied onto the conductive pad.
[Fig. 28] Fig. 28 is a plan view for describing the internal construction of the treatment electrode as shown in Fig. 25.
[Fig. 29] Fig. 29 is a plan view showing another treatment electrode wherein the construction of the electrode connecting section of the treatment electrode as shown in Fig. 27 is modified.
[Fig. 30] Fig. 30 is a structural view schematically showing a still another treatment device which are different from the treatment device as shown in Fig. 1, 6, 10 or 28.
[Fig. 31] Fig. 31 is a structural view schematically showing a further treatment device which are different from the treatment device as shown in Fig. 1, 6, 10, 28 or 30.
[Fig. 32] Fig. 32 is a structural view schematically showing a still further treatment device which are different from the treatment device as shown in Fig. 1, 6, 10, 28, 30 or 31.

### BEST MODE FOR IMPLEMENTING THE INVENTION

Best modes for carrying out the invention will be described with reference to the drawings.
(First Embodiment)

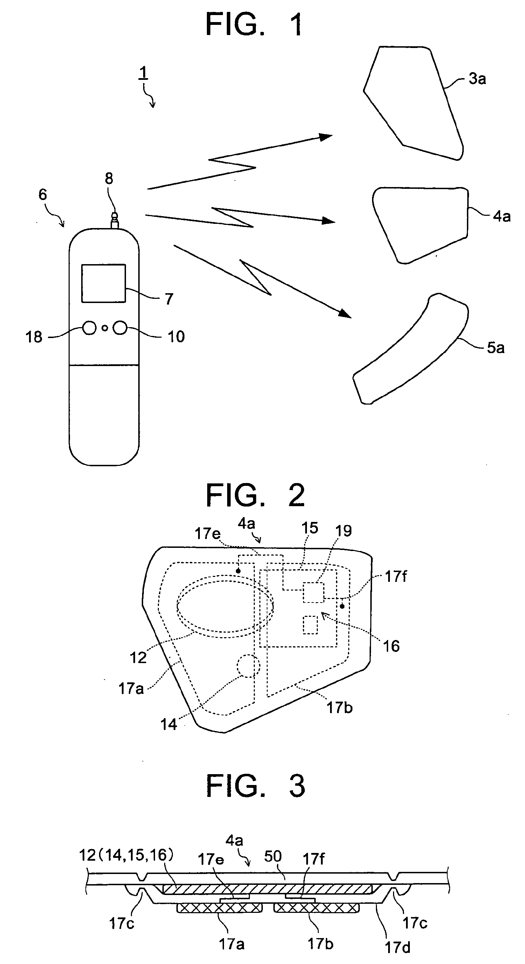

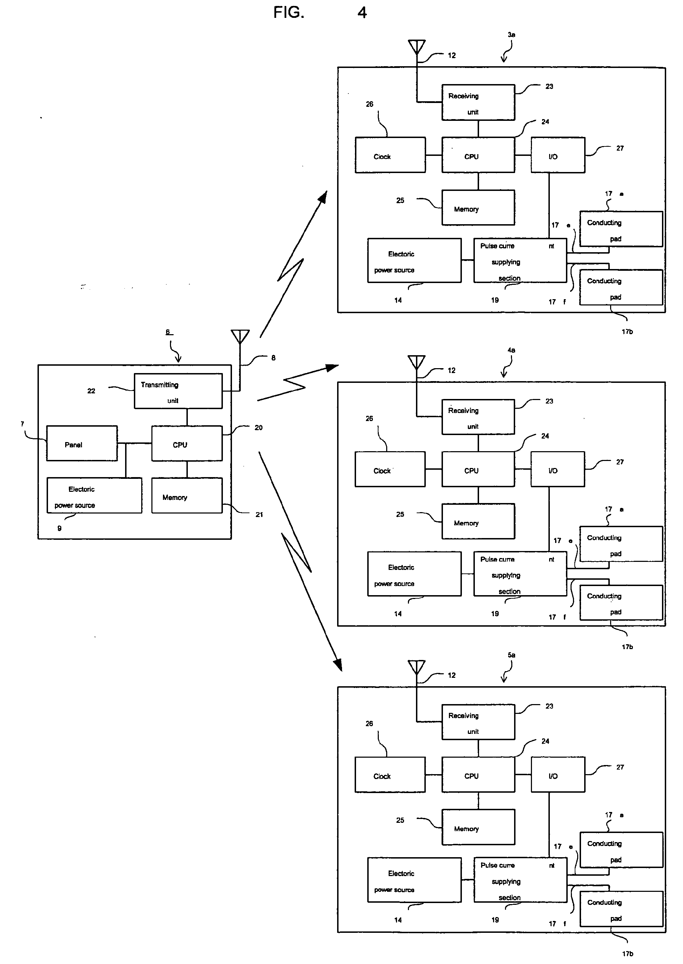

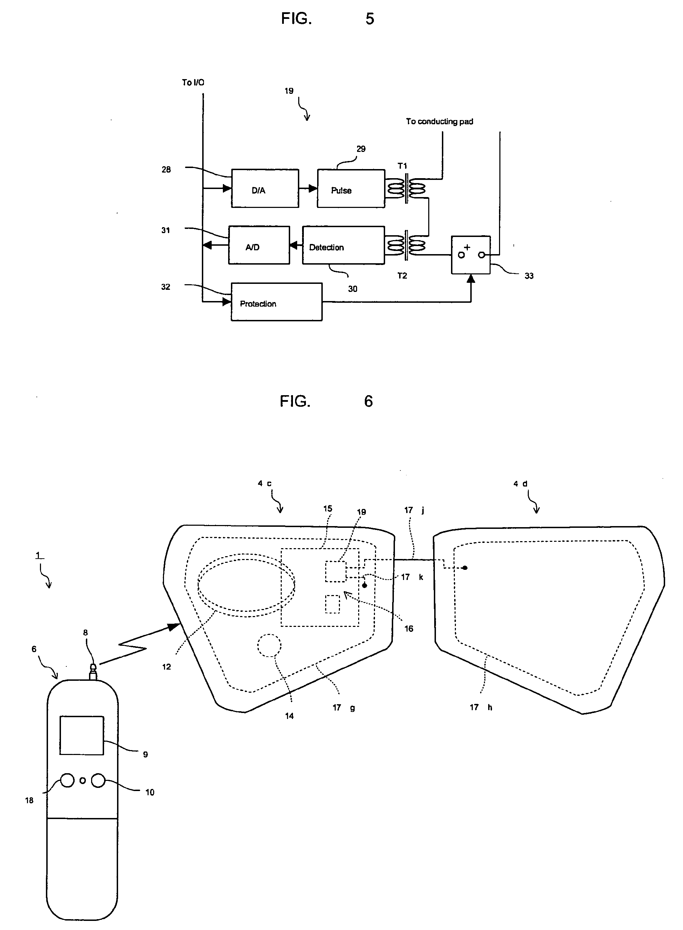

Fig. 1 is a structural view schematically showing a treatment device according to a first embodiment of the present invention, and Fig. 2 is a structural view schematically showing the treatment electrode of the treatment device as shown in Fig. 1, and Fig. 3 is a cross sectional view of the treatment electrode as shown in Fig. 2, and Fig. 4 is a block diagram functionally showing the construction of the treatment device as shown in Fig. 1, and Fig. 5 is a block diagram functionally showing the construction of the pulsed current-supplying section of the treatment device as shown in Fig. 1. As shown in these figures, the treatment device 1 includes treatment electrodes 4a (3a, 5a) to be contacted conductively with a human body and a controller 6 for controlling the treatment electrodes so as to be configured in a cordless pulsed current-type health appliance.

The controller 6 includes an electric power source 9 such as a battery, an electric power source switch 10, a mode setting switch 18 for setting a treatment information, i.e., a treatment mode, a CPU 20 and a memory 21 which function as a control signal generating section for generating a control signal, and a transmission unit 22 and an antenna 8 for transmitting the thus generated control signal at radio transmission, as shown in Figs. 1 and 4. The controller 6 also includes a liquid crystal panel 7 which can display visually the information about the direction of treatment kind, the start and stop of the treatment, the timer setting and the control of the strength of the pulsed current to be supplied.

The CPU 20 of the controller 6 reads out from the memory 21 the treatment kind and the power supplying pattern which are determined on the liquid crystal panel 7 and the mode setting switch 18. Then, the CPU 20 converts the signals about the treatment kind and the power supplying pattern into control signals which are transmitted to the treatment electrodes 4a, 3a, 5a at radio transmission via the transmission unit 22 and the transmission antenna 8. The radio transmission of the control signals may be carried out by using AM wave or FM wave. Instead of the radio transmission, a phase modulated signal or an optical communication using, e.g., infrared light may be employed.

The treatment electrodes 4a (3a, 5a) includes, as shown in Figs. 1 through 4, an electric power source 15 such as a button battery, conducting pads 17a, 17b to be contacted with the skin of the human body in use, a pulsed current supplying section 19 for supplying a pulsed current to a body part located between the conducting pads 17a and 17b via the conducting wires 17e and 17f from the electric power supply 14, a reception unit 23 and antenna 24 which function as a receiver for receiving the transmitted control signal at radio transmission, and chip parts 16 such as a memory 25 and CPU 24 functioning as a controller for the operation of a pulsed current-supplying section 19. The pulsed current-supplying section 19 is connected to the CPU 24 via an I/O 27 and configured so as to alter the output and/or the frequency of the pulsed current. One of the conducting pads 17a and 17b becomes negative (grounded), and the other becomes positive. The intended pulsed current is supplied from the corresponding treatment electrode 4a, 3a or 5a on the basis of the control signal received from the controller 6 at radio transmission. In this case, it is desired that the pulsed currents from the treatment electrodes 4a, 3a and 5a are synchronized in timing, frequency and amplitude. The intended pulsed current may be supplied to the human body with the treatment electrode 4c and/or 4d as shown in Fig. 6. To the treatment electrode 4d is attached one conducting pad 17h, and to the treatment electrode 4c are attached one conducting pad 17g, the antenna 12, the electric power source 14, the chip parts 16 such as the memory 25 and the CPU 24 which are mounted on the controlling board 15, and the pulsed current-supplying section 19. Then, one output of the pulsed current-supplying section 19, e.g., the negative (grounded) output is connected to the conducting pad 17h via the conducting wire 17j and the other output of the pulsed current-supplying section 19, e.g., the positive output is connected to the conducting pad 17g via the conducting wire 17k. In this way, the intended pulsed current can be supplied to the body part of the human body with which the conducting pads 17g and 17h are contacted.

As the treatment operation realizable by the treatment device 1 may be exemplified toning wherein a deeper portion of the human body is stimulated by the pulsed current with a low frequency of 5 to 10 Hz so as to activate the skeletal muscle and thus, promote the blood circulation for the massage of the human body, and drainage wherein a surface portion of the human body is stimulated by the pulsed current with a high frequency of 20 to 100 Hz so as to activate the muscle in the surface region and thus, promote the lymph fluid circulation for the removal of tumor of the human body.

The drainage and the toning can be classified into special drainage and special toning, respectively wherein the pulsed voltage is changed cyclically in amplitude so as to vary the stimulation for the human body. Then, the drainage and the toning can be also classified into time-line drainage and time-line toning wherein the pulsed current is flowed in different electrodes with time and into time-sharing drainage and time-sharing toning wherein the pulsed current is flowed in electrodes simultaneously in time division.

As shown in Fig. 4, each of the treatment electrodes 4a, 3a and 5a receives the control signal transmitted from the controller 6 at radio transmission via the reception antenna 12 and the reception unit 23. The pulsed current supplying section 19 which is provided on each of the treatment electrodes 4a, 3a and 5a reads out from the memory 25 with the CPU 24 the treatment kind and supplying pattern which are indicated by the liquid panel 7 and the mode setting switch 18, inputs the digital trigger obtained from the division of the clock pulse of the standard clock signal generator 26 on the basis of the treatment kind and the supplying pattern into the pulse generator 29 via the interface 27 and the D/A converter 28, and generates a pulse with a given amplitude and frequency to be supplied into the primary side of the transformer T1.

At the pulse current supplying section 19 is provided the transformer T2 in parallel with the transformer T1. In this case, the current detecting circuit 30 is connected to the transformer T2 so as to measure the current and thus, check the excess current on the pulsed current supplying section 19. The detected current at the current detecting circuit 30 is input into the CPU 24 via the A/D converter 31 and the interface 7, and may shut down the current detecting circuit 30 by operating the shut down switch 33 via the current protecting circuit 32 when the detected current is beyond the standard current.

Herein, in this embodiment, the treatment electrode, as shown in Fig. 1, includes various kinds of conducting pads configured so as to match the portions of the human body to be treated. The treatment electrode 3a is for waist sliming, and the treatment electrode 4a is for inner thigh slimming, and the treatment electrode 5a is for hip slimming. The conducting pads 17a and 17b of each electrode are made of gel sheet. In this case, therefore, the conducting pads 17a and 17b can be adhered to the human body intensely without any holding means. In this point of view, the supply efficiency of the pulsed current, i.e., the treatment efficiency can be enhanced easily. In this embodiment, the treatment electrodes 4a, 3a, 5a are employed for the right side of the human body, and other treatment electrodes for the left side of the human body are omitted.

The treatment electrode 4a (3a, 5a),as shown in Fig. 3, includes a base supporter 50 with electric insulation, a flexible base member 17d covering the controlling board 15 on which the chip parts 16 such as the CPU 24 and the memory 25 are mounted and the antenna 12 with the base supporter 50, and the flexible conducting pads 17a, 17b provided on the base member 17d outside. The base supporter 50 is made from flexible synthetic fabric with 18% of polyurethane and 82% of nylon in consideration of the treatment of a user under exercise or the comfortable fit of the base supporter for a user.

The conducting pads 17a and 17b may be made of conductive coating, instead of the gel sheet as mentioned above. In this case, the conducting pad is made as follows. First of all, a base sheet of urethane sheet or the like is prepared, and a conductive material such as conductive carbon ink is formed on the base sheet by means of gravure printing or the like, and the thus obtained conductive sheet is thermally adhered to the base sheet by means of heat press.

An aluminum foil may be adhered to the base member 17d to form a sheet electrode on the base member 17d. The sheet electrode may be made of a composition of matrix of rubber or silicone and metallic power contained in the matrix. The base member 17d may be made of cloth or nonwoven cloth. Since the base member 17d includes an electric insulating region at the periphery thereof, the base member 17d can be secured to the conducting pads 17a and 17b via the insulated region at the periphery with sutures. In this case, since a concentrated current can not be applied to a user from burrs at the secures of the conducting pads 17a and 17b, the user can not suffer from uncomfortable feeling under treatment.

With the treatment device according to this embodiment, when the user inputs a treatment information such as sliming treatment mode into the treatment device via the controller 6, the controlling signal corresponding to the treatment information is transmitted to the treatment electrodes at radio transmission. The treatment electrodes supply pulsed currents to the corresponding conducting pads commensurate with the controlling signal received at radio transmission. According to the treatment device 1 in this embodiment, therefore, since the pulsed currents can be supplied to the human body under cordless, the operationality of parts and instruments the user should handle at treatment can be enhanced remarkably so that the intended treatment such as slimming treatment can be performed easily.

### (Second Embodiment)

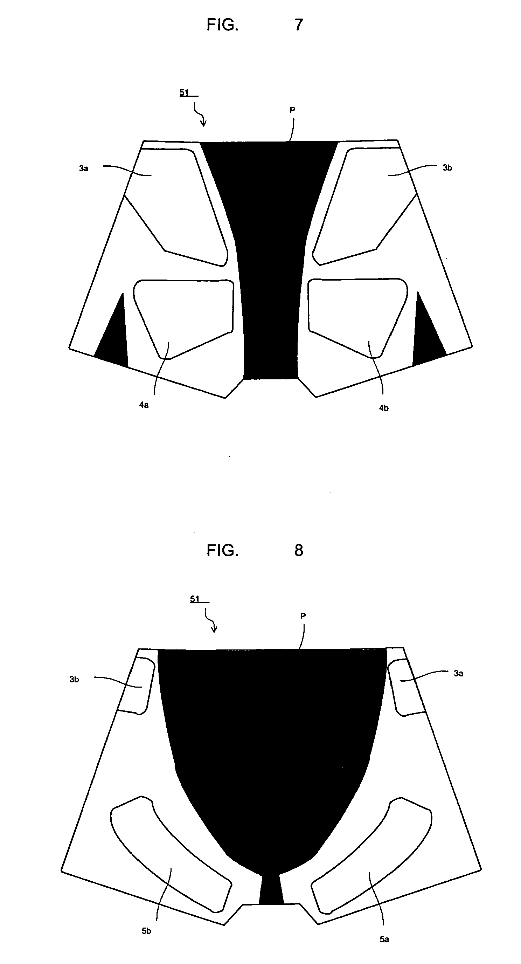

Then, the second embodiment of the present invention will be described with reference to Figs. 7 and 8. Fig. 7 is an elevational view showing a clothing equipped with the treatment electrode according to the second embodiment of the present invention, and Fig. 8 is a backside view of the clothing as shown in Fig. 7.

As shown in these figures, the clothing 51 is to be worn at the lower body. To the clothing 51 are attached the treatment electrodes 3a, 4a, 5a (for the right side of the human body) and the treatment electrodes 3b, 4b, 5b (for the left side of the human body) [which are not shown in First Embodiment] with secures or heat adhesion. To some areas of the clothing 51 are provided power nets P with excellent flexibility which is made from synthetic cloth such as nylon and urethane.

When a user puts the clothing 51 on, the conducting pads 17a, 17b of the treatment electrodes 3a, 3b are contacted to the waist (the rectus abdominis muscle of the user's side and the external abdominal oblique muscle of the lower belly of the user), and the conducting pads 17a, 17b of the treatment electrodes 4a, 4b are contacted to the inner thigh of the user, and the conducting pads 17a, 17b of the treatment electrodes 5a, 5b are contacted to the hip (gluteus maximus muscle). The pulsed current supply to the human body may be carried out by means of the radio transmission from the controller 6 in the same manner as First embodiment.

According to this embodiment, therefore, the user puts the clothing 51 on so that the conducing pads configured so as to match the corresponding parts of the human body can contact to the corresponding treatment portions of the human body. As a result, the setting operation for treatment can be simplified.

### (Third Embodiment)

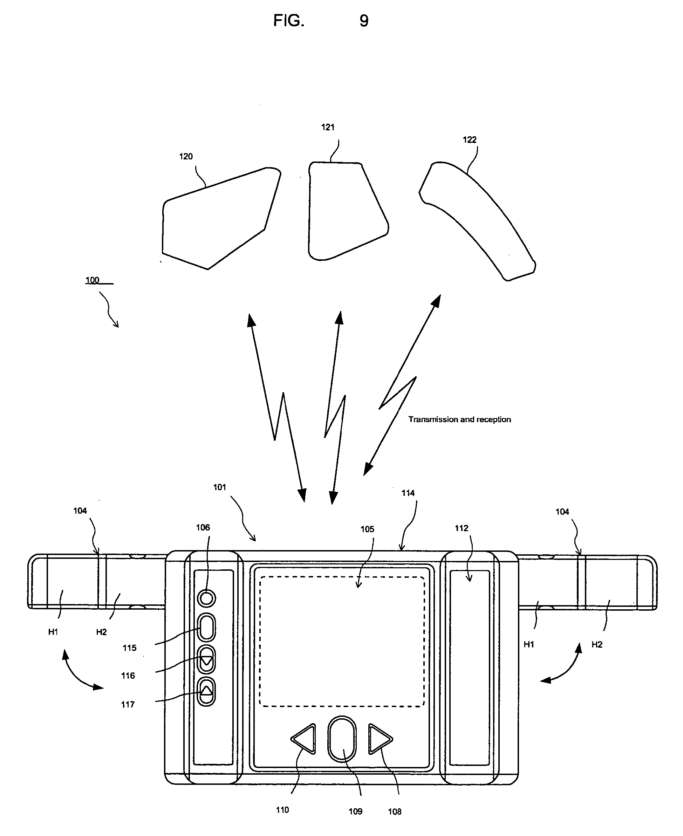

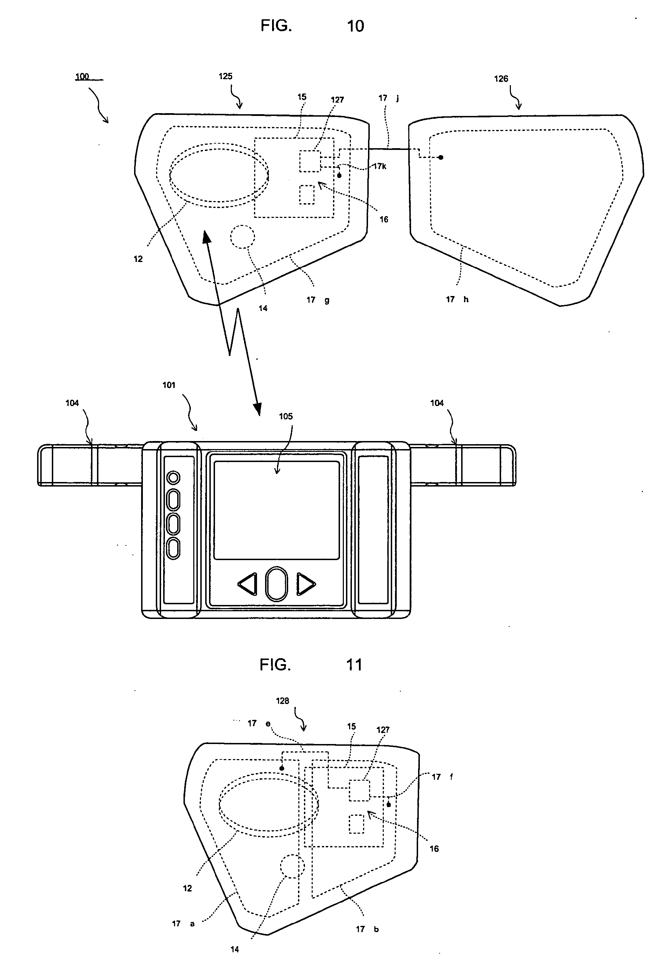

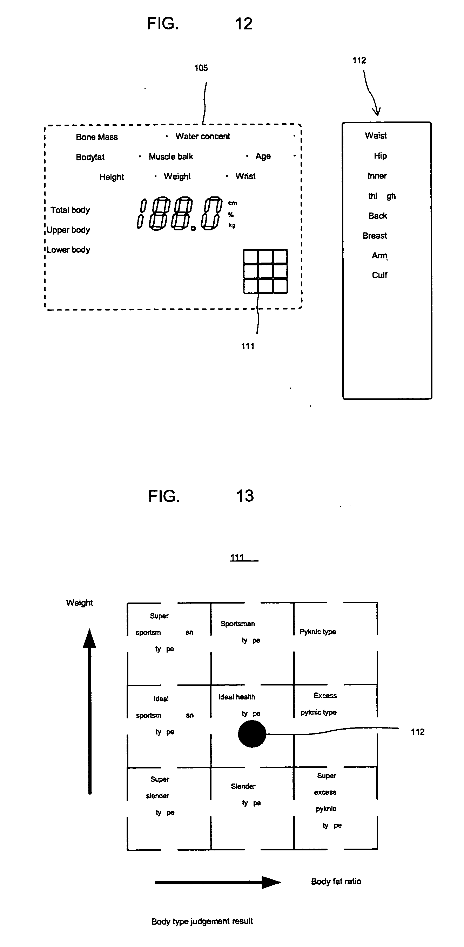

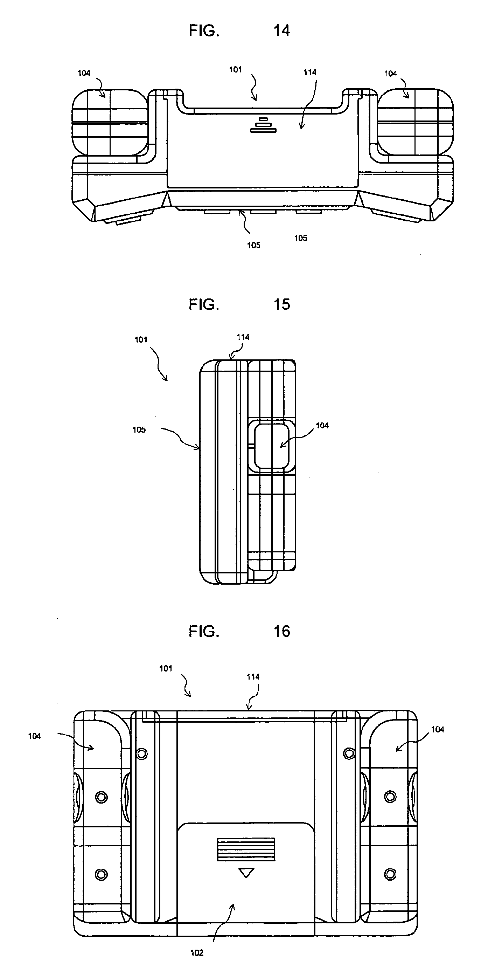

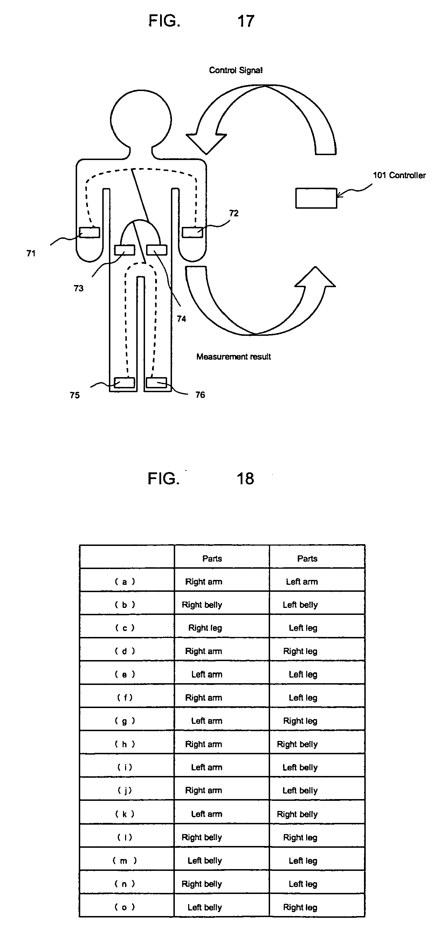

Then, the third embodiment of the present invention will be described with reference to figures. Fig. 9 is a structural view showing a treatment device according to third embodiment of the present invention, and Fig. 10 is a structural view of the treatment electrode equipped to the treatment device as shown in Fig. 9. Fig. 11 is a structural view showing another type of treatment electrode which is different from the treatment electrode as shown in Fig. 10, and Fig. 12 is a structural view showing the liquid crystal displaying section of the controller equipped to the treatment device as shown in Fig. 9. Fig. 13 is a structural view showing the matrix displaying region of the liquid crystal displaying section, and Fig. 14 is a plan view of the controller in Fig. 12. Fig. 15 is a side view of the controller in Fig. 12, and Fig. 16 is a backside view of the controller in Fig. 12, and Fig. 17 is a schematic view showing the entire controlling operation by the treatment device as shown in Fig. 9.

As shown in these figures, the treatment device 100 includes a controller 101 as a base unit and a plurality of treatment electrodes 120, 121, 122......... as cordless handsets. With the treatment device 100 in this embodiment, to the human body are attached a pair of or plural pairs of the treatment electrodes in which measurement currents are flowed so as to measure the impedance of the body part of the human body to which the treatment electrodes are attached. The thus measured impedances at the selected treatment electrodes are transmitted to the controller 101. The controller 101 includes a radio transmission-reception section and a calculation controlling section, and calculates the body fat, the muscle bulk, the bone mass and/or the water content of the body part from the measured impedances which are transmitted. Moreover, the controller 101 may display the calculated the body fat, the muscle bulk, the bone mass and/or the water content of the body part on the displaying device thereof so that a user can select the treatment mode (course) on the displayed results (in this case, the controller 101 may generate a control signal corresponding to the treatment mode selected by the user) . A predetermined pulsed current is flowed into the body part selected by the user via the corresponding treatment electrodes in consideration of the body fat, the muscle bulk, the bone mass and/or the water content. In this way, according to the treatment device of this embodiment, since the treatment mode can be appropriately varied, the user can select the optimum treatment. In the case that the controller 101 requires the impedance measurement (treatment execution) for the treatment electrodes 120, 121, 122..., a given information is transmitted between the controller 101 and the corresponding treatment electrodes in advance so as to render the treatment device 100 under stand-by condition through the initializing process before the measurement. Concretely, the switch-on of the electric power source of the controller 101 is confirmed, and the battery life of the electric power source 14 of the treatment electrode is confirmed through the information transmission.

Then, the controller 101 will be described hereinafter. As mentioned above, the controller 101 can acquire the body fat, the muscle bulk, the bone mass and/or the water content of the body part through the measured impedance through the information transmittance between the controller 101 and the corresponding treatment electrodes. Moreover, the controller 101 can measure the body fat, the impedance and the like by itself. For example, the controller 101 includes a pair of handles 104 which are configured so as to be inserted into and ejected from the sides of the frame of the controller 101. Each of the handles 104 includes the electric current-supplying electrode H1 and the electric current-detecting electrode H2.

As shown in Fig. 9, the liquid crystal displaying section 105 (doubling as the operation section thereof) is provided between the handles 104. Around the liquid crystal displaying section 105 are provided the power source button 106 for on-off switching and the function selecting button 109 (doubling as the controller for the output of the pulsed current) for selecting various functions such as the body fat measurement, the muscle bulk measurement, the bone mass measurement, the water content measurement and/or the treatment mode. Around the liquid crystal displaying section 105 are also provided the reverse button 110 for returning the present displayed data at the displaying section 105 into the previous displayed data thereat, the start/end button 108 for indicating the start and end for a given function and various buttons for inputting personal data such as the gender, age, height, weight, length around the wrist, length around the ankle of a user and setting the strength, frequency, period of time for the intended treatment mode.

As shown in Fig. 16, the electric power source accommodating section 102 is provided at the backside of the frame 114. As shown in Fig. 9, around the liquid crystal displaying section 105 of the controller 101 are provided the electrode selecting buttons 116, 117 for selecting appropriate some treatment electrodes from among the various treatment electrodes, the body part selection displaying section 112 for describing some body parts (e.g., impedance measurement parts and/or pulsed current supplying parts for treatment) selected by the buttons 116, 117 by characters, and the switching button 115 for switching the impedance measurement and the pulsed current supply. The body fat, the muscle bulk, the bone mass and/or the water content of the body part which are calculated from the impedance measurement are displayed at the matrix displaying region 111, as shown in Figs. 12 and 13. In the case that the controller 101 measures the impedance, the body fat and the like by itself, the user selects appropriate operation keys and holds the handles 104 of the controller.

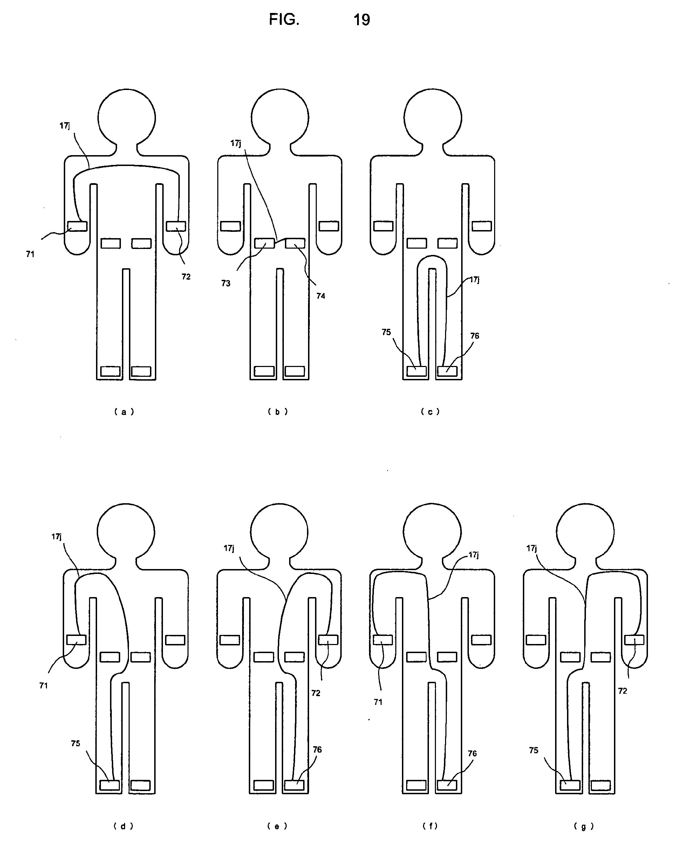

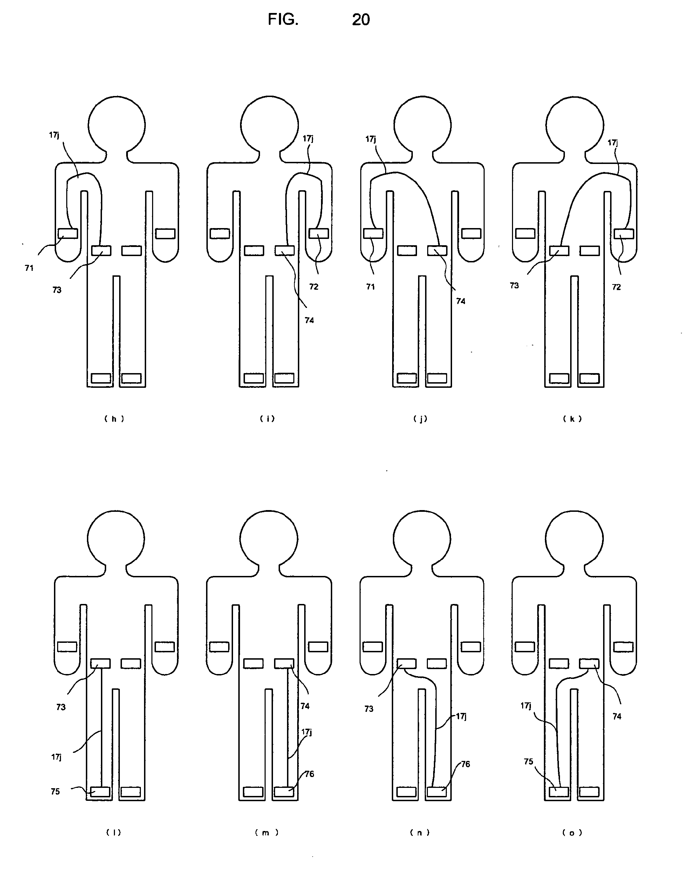

Then, the operation for calculating the body fat, the muscle bulk, the bone mass and/or the water content of the body part through the information transmission between the controller and the corresponding treatment electrodes will be described hereinafter with reference to Figs. 18 to 20, in addition to Figs. 11 to 17. Fig. 18 is a Table relating to the combination of a pair of electrodes among six treatment electrodes which are attached to the human body. Figs. 19 and 20 are schematic views showing some combinations of electrodes on the human body. The selection of electrode attached to the human body as shown in Fig. 18 (a) to (o) corresponds to each schematic view (a) to (o) of Figs. 19 and 20. Concretely, in Figs. 19 and 20 (a) to (o), the impedance measurement, the calculation for the body fat, the muscle bulk, the bone mass and/or the water content and the supply of the pulsed current are carried out at the body part between the electrodes connected by the conductive wire 17j.

As shown in Fig. 10, the treatment electrode 125, 126... are formed in the same manner as the treatment electrodes 4c, 4b... as shown in Fig. 6 relating to the first embodiment. In this embodiment, however, the pulsed current supplying section 127 (refer to Fig. 10) doubling as a pulsed current supplier for impedance measurement is provided, instead of the pulsed current supplying section 19 (refer to Fig. 6) which the electrode 4c includes. In this case, the treatment electrode 128 as shown in Fig. 11, which is formed in the same manner as the treatment electrode 4a as shown in Fig. 2, may be employed. Herein, it is desired that the pulse width of a pulsed current output from the pulsed current supplying section 127 is set within 100-500 µsec. Moreover, the pulsed current supplying section 127 is configured so as to generate a pulsed current within middle range frequency through high range frequency.

The treatment electrodes 71 to 76 as shown in Fig. 17 are attached to both arms, the lower belly so as to sandwich the umbilicus or both legs at the impedance measurement. In this case, the impedance of the upper body, the lower body or the belly can be measured concisely. The user to be measured in impedance is requires to take a given stance. Since the measurement impedance is changed on the measurement distance, the measurement distance is set so as to measure the impedance within a range of 100 to 3000 Ω. As shown in Figs. 18, 19 and 20, a pulsed current of 800 µA is supplied to the body part from the pair of (two) electrodes selected from among six electrodes. It is desired that the frequency of the pulsed current is set to 50 Hz. The pulse width is set wider for fat user, and narrower for thin user. Also, the number of heart beat is detected so that the change of the heart rhythm can be detected.

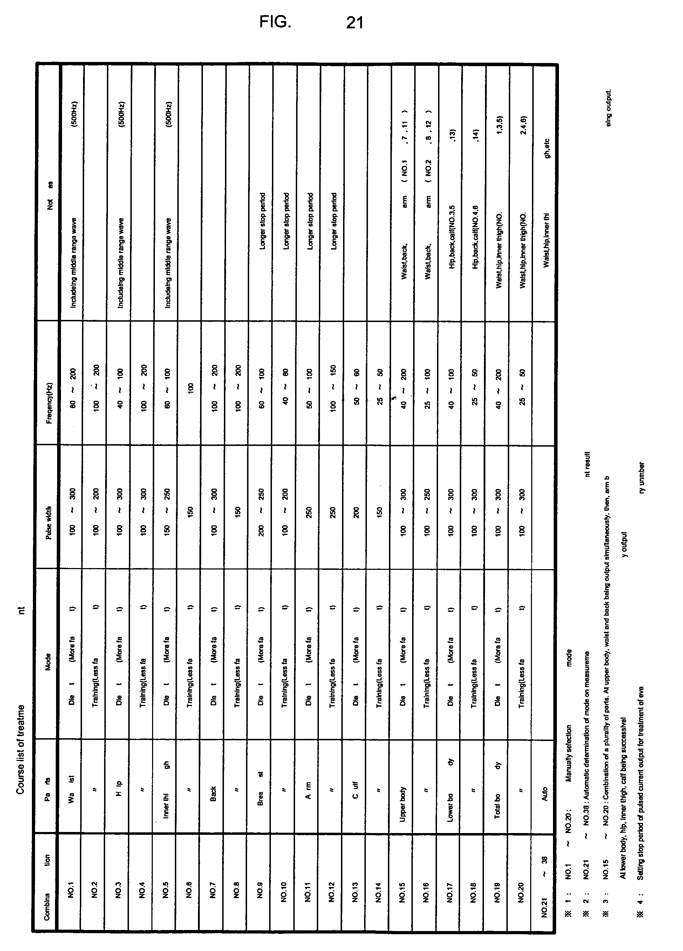

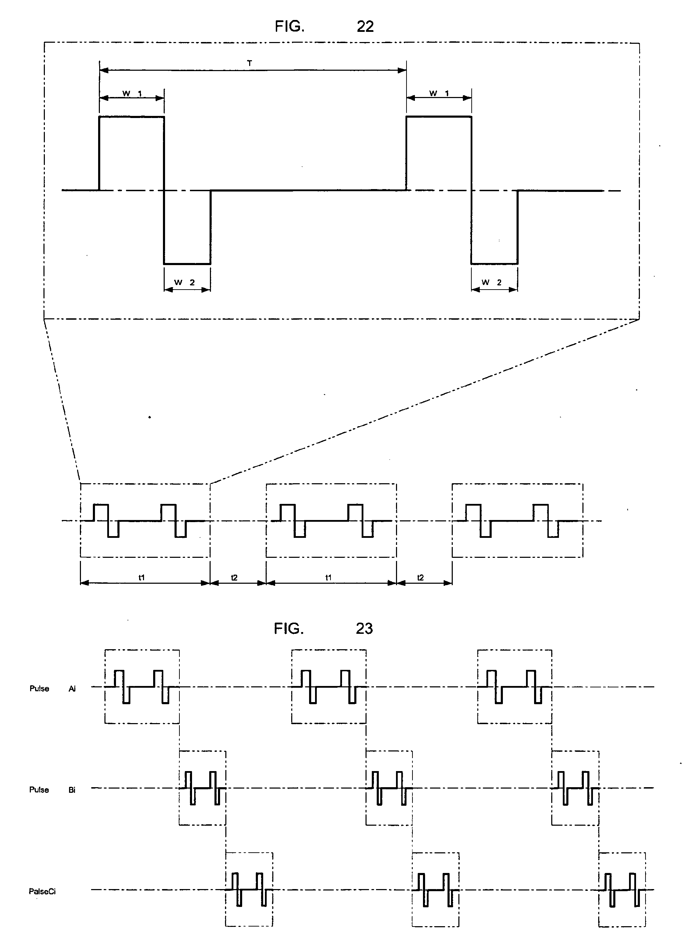

The various treatment courses using the treatment device 100 in this embodiment will be described with reference to Figs. 21 to 23. Fig. 21 is a Table showing treatment courses carried out by the treatment device 100. Fig. 22 is a schematic view showing the wave form of a pulsed current to be supplied to the human body through the treatment electrodes of the treatment device 100. Fig. 23 is a schematic view showing the wave forms of pulsed currents to be supplied to the human body through the treatment electrodes of the treatment device 100.

As shown in Fig. 21, with the treatment device 100 in this embodiment, 20 treatment courses which the user manually selects are prepared, and 18 treatment courses which the treatment device 100 automatically selected are prepared. In this embodiment, therefore, total 38 treatment courses are prepared for the treatment device 100. In this case, the manual selection means that the user selects a given treatment course referring to the body fat, the muscle bulk, the bone mass and/or the water content of the body part which are displayed on the liquid crystal displaying section 105 (which are calculated on the impedance measurement results) (refer to Fig. 21, *1). The automatic selection means that the treatment device 100 automatically selects a given treatment course on the calculated result for the body fat, the muscle bulk, the bone mass and/or the water content, and then, conducts some operations successively (refer to Fig. 21, *2).

Concretely, the 20 manual courses, designated by the reference numerals 1 to 20, are classified into the following courses. First of all, seven treatment courses may be exemplified. With the seven treatment courses, the treatment electrodes are attached to seven parts such as waist, hip, inner thigh, back, breast, arm, calf, for example, and each body part as exemplified above will be treated. Moreover, three treatment courses may be exemplified. With the three treatment courses, three parts are selected from among the seven parts to which the treatment electrodes are attached as described above, and conducted multiply. The user selects one treatment course from among the above-mentioned ten treatment courses and selects a diet mode or a training mode [(7 + 3) × 2 = 20 courses] referring to the body fat and the like on the treatment course. Herein, the diet mode means a treatment mode for intending the fat-burning, and the training mode means a treatment mode for intending the muscle-building. The 18 courses designated by reference numerals 21 to 38 may be appropriately changed on the impedance measurement result of the body part (or body fat calculation result).

In detail, the courses designated by No. 1, No. 3 and No. 5 render the frequencies of the pulsed currents to be supplied to the human body within 60 to 200 Hz, 40 to 100 Hz and 60 to 100 Hz, respectively, but the upper limited frequency of the pulsed current may be increased to a middle range frequency of 500 Hz when the course is applied for the waist, hip or inner thigh which contains more fat. Then, as shown in Fig. 22, in all of the treatment courses, the output of the pulsed current, which is supplied to the human body via the treatment electrodes, is shaped such that the pulsed current output period t1 and the pulsed current stop period t2 are repeated (refer to Fig. 21, *4). In this way, since the pulsed current stop period is formed, the muscle of the human body can be rest so that the muscle training efficiency during the successive output period t11 can be developed. Herein, the stop periods t2 of the treatment courses designated by No. 9 to 12 for breast or arm may be set longer than the stop periods t2 of the treatment courses for another part.

The minimum pulse width of the pulsed current to be supplied to the human body from the treatment electrodes is set to 100 µsec, and the pulse widths W1 and W2 of the pulsed currents may be increased in accordance with the body fat (the pulse width being enlarged at more body fat and the pulse width being narrowed at less body fat). It is desired that the pulse width is set in consideration of the gender and body fat of the user. The body fat percentages may be classified into the ranges not more than 20%, 20 to 26%, 26 to 30% and not less than 30%. Then, as shown in Fig. 22, the frequency of the pulsed current to be supplied to the human body from the treatment electrodes is set within a range of 50 to 100 Hz when the body fat is lower so that the distance between the surface skin and the muscle is shorter (it being estimated that the distance between the surface skin and the muscle is set within a predetermined distance). Then, the frequency of the pulsed current to be supplied to the human body from the treatment electrodes is set within a range of 1 to 10 Hz when the body fat is higher so that the distance between the surface skin and the muscle is longer (it being estimated that the distance between the surface skin and the muscle is set beyond a predetermined distance). The pulsed current with a higher frequency of not less than 100 Hz can bring about the higher diet effect so as to be frequently employed for the diet mode. Then, in order to enhance the diet effect, it is desired that the output of the pulsed current is increased.

The treatment courses designated by No. 15 to 20 are applied for the combination of three parts of the human body. For example, with the upper body treatment relating to No. 15 and No. 16, pulsed currents are applied to the treatment electrodes which are attached to the waist and the back of the human body, and then, another pulsed current is applied to the treatment electrode which are attached to the arm. With the upper body treatment relating to No. 17 and No. 18, as shown in Fig. 23, pulsed currents are successively applied to the treatment electrodes which are attached to the hip (pulsed current Ai), the inner thigh (pulsed current Bi) and the calf (pulsed current Ci)(refer to Fig. 21, *3).

With the treatment course relating to No. 17 and 18, a given information is transmitted between the controller 101 and the corresponding treatment electrodes A, B, C (see, Fig. 24) in advance so as to render the controller 101 and the treatment electrodes under stand-by condition through the initializing process before the measurement. Then, as shown in Fig. 24, the impedance measurement requirement A1, the impedance measurement result return A2, the measurement requirement B1, the measurement result return B2, the measurement requirement C1 and the measurement result return C2 are carried out between the controller 101 and the corresponding treatment electrodes A, B, C. Thereafter, the controller 101 calculates the body fat, the muscle bulk, the bone mass and/or the water content on the measured impedance results at the hip, inner thigh and calf, and then, supplies the pulsed currents (conducts treatment courses) to the hip, the inner thigh and the calf via the treatment electrodes A, B, C, respectively.

Concretely, with the treatment device 100, as shown in Fig. 23, the pulsed current Ai supply requirement A3, the pulsed current Ai supply completion notice A4, the pulsed current Bi supply requirement B3, the pulsed current Bi supply completion notice B4, the pulsed current Ci supply requirement and the pulsed current Ci completion requirement are carried out between the controller 101 and the corresponding treatment electrodes A, B, C. The above-described process will be repeated. When the pulsed currents are supplied to the treatment electrodes A, B, C at predetermined times, the pulsed current supply is completed so that the treatment courses No. 17 and 18 are completed.

Therefore, according to the treatment device 100 of this embodiment, since the treatment mode can be appropriately controlled in accordance with the body fat, the muscle bulk, the bone mass and/or the water content which are calculated on the measured impedance of the body part, the user can select the appropriate treatment course easily at radio transmission.

### (Fourth Embodiment)

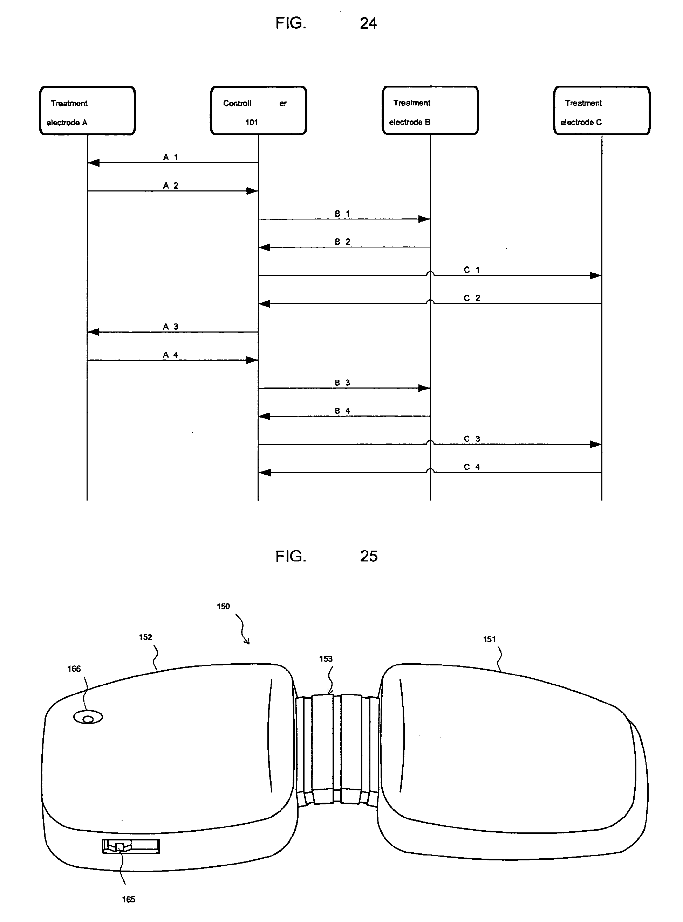

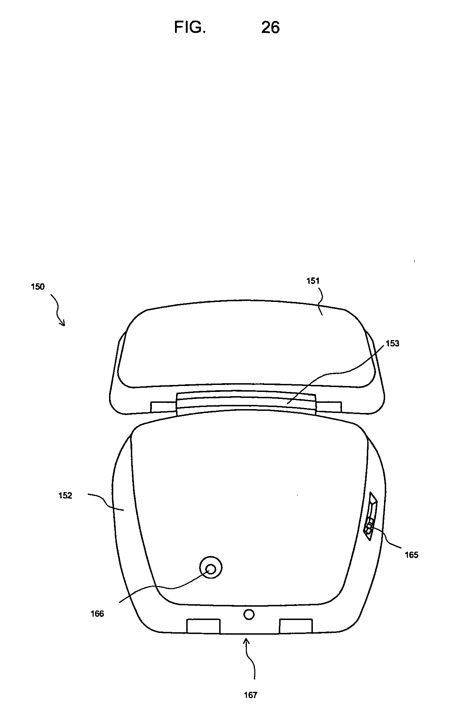

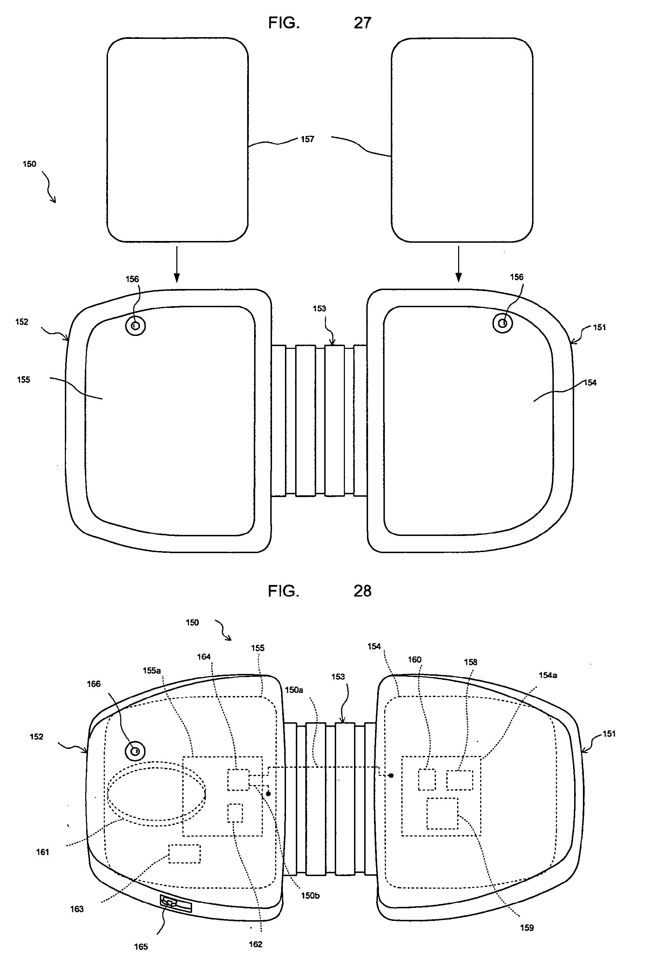

Then, the fourth embodiment of the present invention will be described with reference to figures. Fig. 25 is a perspective view showing the treatment electrode of a treatment device according to a fourth embodiment of the present invention, and Fig. 26 is a perspective view showing the treatment electrode as shown in Fig. 25, as viewed from the terminal for electric charge. Fig. 27 is a plan view showing the treatment electrode as viewed from the conductive pad and showing the gel sheet applied onto the conductive pad, and Fig. 28 is a plan view for describing the internal construction of the treatment electrode as shown in Fig. 25.

As shown in Figs. 25 to 28, with the treatment electrode 150, the practical utility is enhanced in comparison with the practical utility for the treatment electrodes 125, 126 in the third embodiment so that a given treatment information can be transmitted and received between the controller 101 and the treatment electrode 150 at radio transmission as shown in Fig. 10. The treatment electrode 150 mainly includes a pair of electrode bodies 151, 152 and the electrode connector 153 for electrically and mechanically connecting the electrode bodies 151, 152.

With the electrode bodies 151, 152, as shown in Figs. 25 and 26, the frame is formed through the molding of ABS (acrylonitrile butadiene styrene) resin or the like.. At the bottom surfaces of the electrode bodies 151, 152 are provided the conducting pads 154, 155 with the screws 156 so as to be projected from the frame slightly, as shown in Fig. 27. The conducting pads 154, 155 are configured so as to be formed from the ABS resin core and the chrome plating film covering the core, for example. The treatment electrode 150 includes the sealing areas on which the gel sheets 157 with an electric conduction and an adhesion to the skin of the human body are disposed. In this case, the adhesion between the skin of the human body and the conducting pads 154, 155 can be enhanced without any special adhering member. The gel sheet is exchangeable so that a plurality of gel sheets can be prepared. Therefore, dirty gel sheet subject to treatment processes can be exchanged easily.

As shown in Fig. 28, into one electrode body 151 is accommodated the mainly digitalized signal processor including the memory 158 for storing an electric power supply pattern in accordance with the treatment kind, the CPU 159 for conducting various calculation, the wave form outputting circuit 160 for shaping and outputting the signal divided from the standard clock signal on the electric power supply pattern read out of the memory 158 with the CPU 159, and the like. The signal processor is mounted on the control board 154a.

As shown in Fig. 28, onto the other electrode body 152 are mounted the mainly analogical signal processor including the antenna 161 for conducting the radio transmission for the controller 101, the communication controlling circuit 162, which is mounted on the controlling board 155a, for controlling the operation of the antenna 161, the rechargeable battery 163, the pulsed current supplying circuit 164 for supplying the pulsed currents for treatment, which are generated and amplified through the D/A conversion and transform of the output from the wave form outputting circuit 159, to the conducting pads 154, 155 of the electrode bodies 151, 152. In this way, the digital signal processor and the analog signal processor are electrically connected with one another, but mounted on the corresponding electrode bodies 151 and 152, separately, so that the electrical interference between the signal processors can be prevented and thus, the signal processing can be stably conducted.

Then, as shown in Figs. 25 and 26, at the outer sides of the electrode body 152 are provided the slide switch 165 for switching the on/off of the electric power source, the LED lamp 166 for visually displaying the on/off condition of the electric power source which is switched by the slide switch 165 and the charging terminal 167 for connecting the charging adaptor to the rechargeable battery 161.

In the electrode connector 153 is formed the through-hole for inserting the wire to electrically connect the electrode bodies 151, 152. Namely, in the through-hole are accommodated the wire (not shown) for connecting the wave form outputting circuit 160 and the pulsed current supplying circuit 164 and the wire 150a for connecting the conducting pad 154 of the electrode body 151 and the pulsed current supplying circuit 164. Herein, one output, e.g., the negative (grounded) output is connected to the conducting pad 154 via the wire 150a, and the other output, e.g., the positive output is connected to the conducting pad 155 via the wire 150b. Therefore, the pulsed current can be supplied to the body part of the human body by contacting the conducting pads 154, 155.

Moreover, the electrode connector 153 to connect the electrode bodies 151 and 152 is made of a flexible material such as rubber so as to be flexible. Since the electrode connector 153 can be deformed commensurate with the convex-concave surface shape of the human body, the conducting pads 154, 155 of the electrode bodies 151, 152 which are provided at both sides of the electrode connector 153 can be attached to the surface region of the human body steadily so that the intended treatment process can be conducted.

According to the treatment electrode 150 of this embodiment, since various wires to connect the pair of conducting pads are covered completely with the electrode connector 153 so that the entire electrode structure can be unified, the portability of the treatment electrode can be developed and the handling of the treatment electrode for attaching by the user can be simplified.

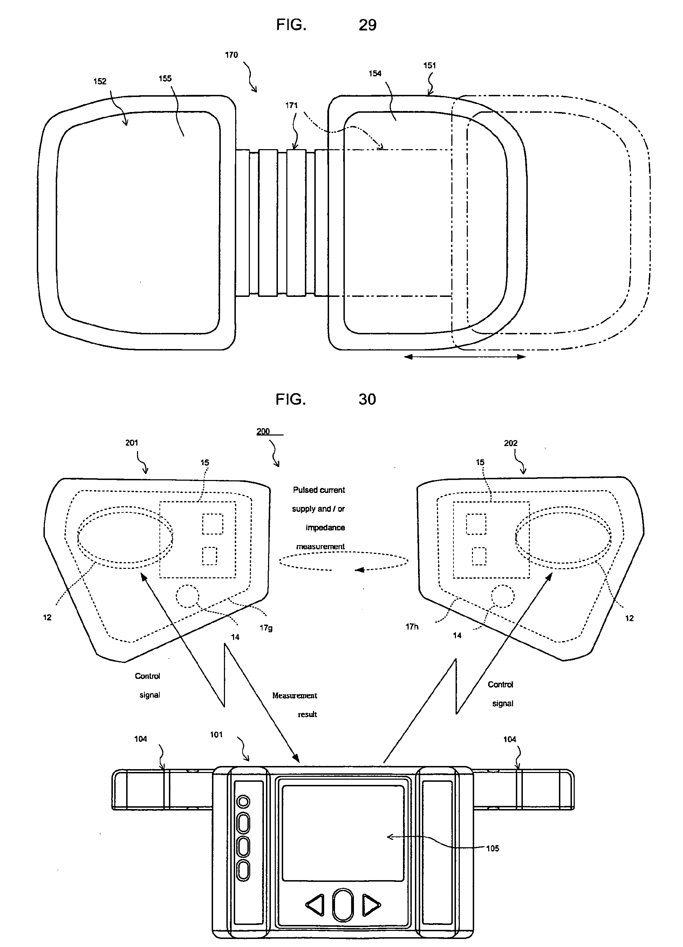

Herein, as shown in Fig. 29, the length of the electrode connector 171 for electrically and mechanically connecting the electrode pads 154, 155, i.e., the electrode bodies 151, 152 can be controllable so as to form the treatment electrode 170. In this case, since the distance between the conducting pads 154, 155 can be easily controlled with the treatment electrode 170, the position of the conducting pads on the human body can be easily shifted.

Although the present invention was described in detail with reference to the above examples, this invention is not limited to the above disclosure and every kind of variation and modification may be made without departing from the scope of the present invention. For example, instead of the pair of treatment electrodes 125, 126 being connected with the wire 17j at fixed line as the treatment device 100 exemplified in Fig. 10, the pair of electrodes 201, 202 to be attached to the human body is connected at radio transmission so that the control signal for supplying the pulsed current and/or the signals containing the impedance measurement requirement signal and impedance measurement result signal can be transmitted to the treatment electrodes 201, 202 from the controller 101 at radio transmission so as to form the treatment device 200.

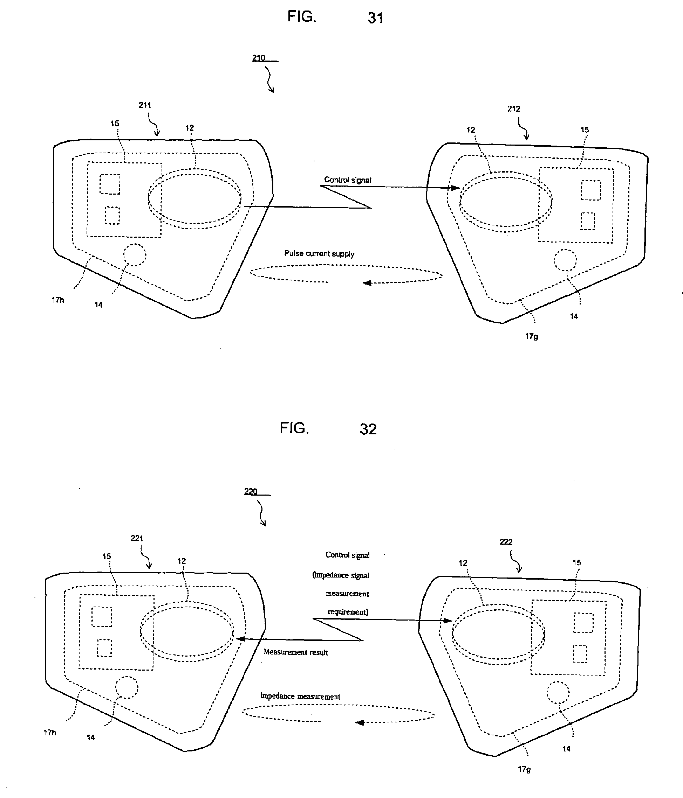

Moreover, as shown in Fig. 31, the treatment electrode 211 is formed so as to contain the function of the controller 101, and the treatment electrodes 211 and 212 are connected via radio transmission so that the control signal for supplying the pulsed current can be transmitted between the treatment electrodes 211, 212 at radio transmission, so as to form the treatment device 210.

In addition, as shown in Fig. 32, the treatment electrodes 211 and 212 are connected via radio transmission so that the signals containing the impedance measurement requirement signal and the impedance measurement result signal can be transmitted between the treatment electrodes 211, 212 at radio transmission, so as to form the treatment device 220. Furthermore, the treatment device which can conduct the pulsed current supply and the impedance measurement may be provided.

### INDUSTRIAL APPLICABILITY

The present invention can be applied widely in electronic and electric equipment manufacturing field.

## Claims

1. An electrode for treatment, comprising:
an electric power source;
a conducting pad for attaching a human body;
a treatment current supplying means for supplying a pulsed current to a body part to which said conducting pad is attached on the electric power supply from said electric power source;
a receiving means for receiving an external control signal at radio transmission; and
a controlling means for controlling said treatment current supplying means on the basis of said control signal received by said receiving means at radio transmission.

2. The treatment electrode according to claim 1, wherein said treatment current supplying means is configured so as to stop the supply of said pulsed current at a given period.

3. The treatment electrode according to claim 1, wherein said conducting pad is comprised of a plurality of conducting pads.

4. The treatment electrode according to claim 3, further comprising a conducting pad connector for electrically and mechanically connecting said plurality of pads, wherein the length of said conducting pad connector is changeable.

5. The treatment electrode according to claim 3, further comprising a conducting pad connector for electrically and mechanically connecting said plurality of pads, wherein said conducting pad connector is flexible.

6. The treatment electrode according to claim 1, further comprising an impedance measuring means for measuring the impedance of said body part to which said conducting pad is attached by flowing a measuring current in said body part.

7. The treatment electrode according to claim 6, further comprising an impedance information transmitting means for transmitting a measurement result in impedance by said impedance measuring means outside at radio transmission, wherein said receiving means receives, as said control signal, treatment controlling information corresponding to the body fat, the muscle bulk, the bone mass and/or the water content of said body part which are calculated from the measured impedances.

8. The treatment electrode according to claim 7, wherein said controlling means varies the output and/or frequency of said pulsed current to be supplied to said human body by said treatment current supplying means on, as said control signal, said treatment controlling information corresponding to the body fat, the muscle bulk, the bone mass and/or the water content of said body part.

9. The treatment electrode according to claim 8, wherein said controlling means varies the width of said pulsed current commensurate with the degree of the body fat of said body part which is calculated.

10. The treatment electrode according to claim 1, further comprising a pad adhering means for adhering said conducting pad to said human body.

11. The treatment electrode according to claim 10, wherein said pad adhering means is configured such that said conducting pad is made of an adhesive sheet with electric conduction.

12. The treatment electrode according to claim 1, wherein said conducting pad is comprised of a plurality of conducting pads commensurate with different kinds of shapes of said body part.

13. The treatment electrode according to claim 12, further comprising a clothing with said plurality of conducting pads which are fixed so as to be contacted to said body part at treatment.

14. The treatment electrode according to claim 1, further comprising a number of heart beat detecting means for detecting the number of heart beat through said conducting pad to be contacted to said body part.

15. A device for treatment, comprising:
a treatment electrode; and
a controller for controlling said treatment electrode,
said controller including:
an input means for inputting information about said treatment;
a control signal generating means for generating a control signal on said information input by said input means; and
a control signal transmitting means for transmitting said control signal generated by said control signal generating means at radio transmission.

16. The treatment device according to claim 15, further comprising a plurality of conducting pads.

17. The treatment device according to claim 15, wherein said controller further includes a pulsed current supply selecting means which is configured so as to switchably select a pair of conducting pads or a pair of treatment electrodes for supplying a pulsed current through a treatment current supplying means from among three or more conducting pads or treatment electrodes.

18. The treatment device according to claim 15, wherein said pulsed current supply selecting means is configured so as to switchably and successively select said pair of conducting pads or said pair of treatment electrodes so that said pulsed current is supplied successively through the successively selected pair of conducting pads or treatment electrodes.

19. The treatment device according to claim 15, wherein said treatment electrode includes an impedance measuring means for measuring the impedance of said body part to which said conducting pad is attached by flowing a measuring current in said body part, and said controller includes a selecting means which is configured so as to switchably select a pair of conducting pads or a pair of treatment electrodes for measuring an impedance of said body part through an impedance measuring means from among three or more conducting pads or treatment electrodes.

20. The treatment device according to claim 15, wherein said treatment electrode further includes an impedance measuring means for measuring the impedance of said body part to which said conducting pad is attached by flowing a measuring current in said body part, and
said controller further includes:
an impedance information receiving means for receiving, at radio transmission, an impedance measurement result of said body part which is measured by said impedance measuring means;
a calculating means for calculating the body fat, the muscle bulk, the bone mass and/or the water content of said body part on the measured impedance received by said impedance information receiving means;
a control signal generating means for generating treatment information as a control signal on the calculated result by said calculating means; and
a control signal transmitting means for transmitting, at radio transmission, said control signal generated by said control signal generating means.
